# EUROPÄISCHE PATENTSCHRIFT

(11) **EP 1 731 107 B1**
(45) Veröffentlichungstag und Bekanntmachung des Hinweises auf die Patenterteilung: **29.02.2012**
(21) Anmeldenummer: 06019615.1
(22) Anmeldetag: 26.08.2003
(51) Int. Cl.: A61B 17/80

(54) **Bohrbüchse zur Verwendung in einer Knochenplatte**
Drill bushing for use in a bone platte
Canon de perçage à utiliser dans une plaque d'ostéosynthèse

(43) Veröffentlichungstag der Anmeldung: 13.12.2006
(62) Teilanmeldung aus: 03818256.4
(73) Patentinhaber: Synthes GmbH, 4436 Oberdorf (CH)
(72) Erfinder: Schneider, Rolf, 2562 Port (CH)
(74) Vertreter: Höhfeld, Jochen

(56) Entgegenhaltungen:
- EP-A- 0 530 585
- WO-A-00/66012
- WO-A-97/09000
- DE-A- 4 343 117
- FR-A- 742 618

## Beschreibung

Die Erfindung bezieht sich auf eine Bohrbüchse zur Verwendung in einer Knochenplatte gemäß dem Oberbegriff des Patentanspruchs, wobei die Knochenplatte gemäß Oberbegriff des Patenansprüchs aus der DE-A-4 943 117 bekannt ist.

Solche Knochenplatten eigenen sich für Indikationen am gesamten Skelett. Von besonderer Bedeutung sind aber die üblichen Groß- und Kleinfragment-Indikationen für die operative Knochenbruchbehandlung.

Bohr- oder Führungsbüchsen zum Vorbohren von Löchern im Knochen oder zum Einbringen von Kirschner-Drähten werden im Stand der Technik, wie z.B. aus der FR-A-742618 bekannt, in die Plattenlöcher eingeschraübt.

Gemäß der vorliegenden Erfindung, wie sie im Patentanspruch definiert ist, können mindestens drei Ausnehmungen im Plattenloch zur Führung von Bohr- oder Führungsbüchsen verwendet werden. Die Bohr- oder Führungsbüchsen brauchen dabei nicht mehr - wie beim Stand der Technik - in die Plattenlöcher eingeschraubt zu werden, sondern können, dank der Ausnehmungen, lediglich in die Plattenlöcher eingesteckt werden, was auf eine einfache Art Zentrum und Richtung der Lochachse ergibt, ohne dabei die Schraubenauflager zu verletzen. Die kannulierten Bohr- oder Führungsbüchsen brauchen dazu an ihrer Spitze lediglich die Negativform der Plattenloch-Geometrie aufzuweisen, ohne irgendwelche Gewinde oder sonstige, gleichwirkende Strukturen. Gegebenenfalls kann auch ein Schnappmechanismus damit verbunden werden.

Die Ausnehmungen können sich von der Oberseite zur Unterseite hin zylindrisch oder konisch erstrecken.

Die Ausnehmungen können sich über die gesamte Höhe der Knochenplatte von der Oberseite bis zur Unterseite hin erstrecken.

Im Nachfolgenden werden Ausführungsbeispiele der Knochenplatte näher erläutert, bei welchen die Bohrbüchse gemäß der Erfindung verwendet werden kann.

Es zeigen:
Fig. 1 einen Längsschnitt durch eine Knochenplatte mit konischen Plattenlöchern;
Fig. 2 einen Längsschnitt durch eine Knochenplatte mit sphärischen Plattenlöchern;
Fig. 3 eine Aufsicht auf eine Knochenplatte mit 3 Ausnehmungen in der Innenmantelfläche der Plattenlöcher;
Fig. 4 eine Variante der Knochenplatte nach Fig. 3 mit stärkeren Ausnehmungen in der Innenmantelfläche der Plattenlöcher;
Fig. 5 eine Aufsicht auf eine Knochenplatte mit Gewindeeinsätzen mit 4 Ausnehmungen in der Innenmantelfläche elliptisch ausgebildeter Plattenlöcher;
Fig. 6 eine perspektivische Ansicht einer Knochenplatte nach Fig. 1 von oben mit eingesetzten Knochenschrauben;
Fig. 7 eine perspektivische Ansicht einer Knochenplatte nach Fig. 1 von unten mit eingesetzten Knochenschrauben;
Fig. 8 einen Längsschnitt durch eine Knochenplatte mit darin eingesetzter Knochenschraube ohne Abwinkelung ; und
Fig. 9 einen Längsschnitt durch eine Knochenplatte mit darin eingesetzter Knochenschraube mit Abwinkelung.

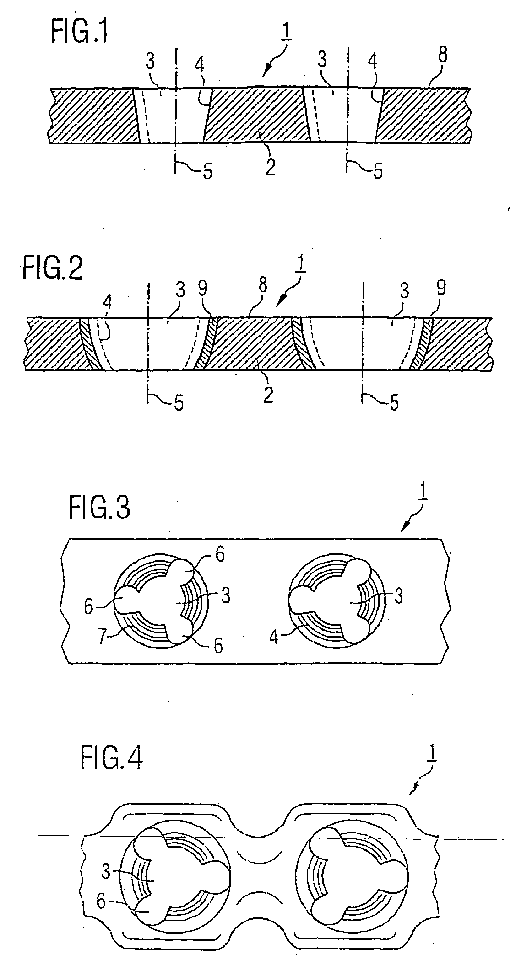

Die in den Fig. 1 und 3 dargestellte Knochenplatte 1 besitzt eine knochenseitige Unterseite 2, eine Oberseite 8 und mehrere, die Unterseite 2 mit der Oberseite 8 verbindende, Plattenlöcher 3 mit einer zentralen Lochachse 5. Die Plattenlöcher 3 weisen eine sich gegen die Unterseite 2 hin konisch verjüngende Innenmantelfläche 4 auf. Im Weiteren weist die Innenmantelfläche 4 drei sich radial von der Lochachse 5 weg erstreckende Ausnehmungen 6 in einem regelmäßigen Abstand von 120° auf. Ihre periphere Ausdehnung beträgt ca. 40° und sie verlaufen ausschließlich im Bereich der Innenmantelfläche 4. Die Ausnehmungen 6 erstrecken sich über die gesamte Höhe der Knochenplatte 1 von der Oberseite 8 bis zur Unterseite 2 hin konisch. Die Innenmantelfläche 4 ist zudem mit einer dreidimensionalen Strukturierung 7 in Form eines Gewindes versehen.

In Fig. 4 ist eine Variante der Ausführung nach Fig. 3 dargestellt, bei welcher sich die Ausnehmungen radial von der Lochachse weg über den Bereich der Innenmantelfläche hinaus erstrecken.

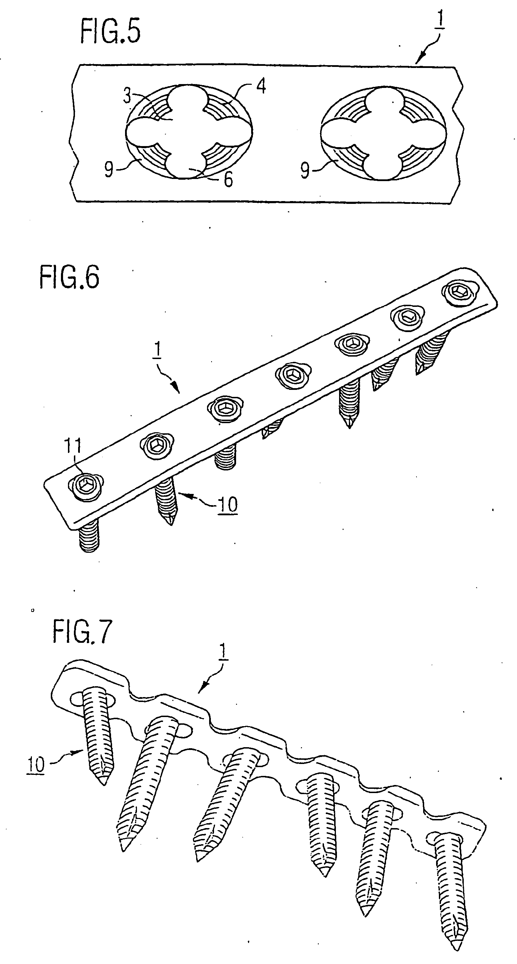

In den Fig. 2 und 5 ist eine weitere alternative Ausführungsform dargestellt, bei welcher die Plattenlöcher 3 als Langlöcher ausgebildet sind. Die Knochenplatte besteht zur Hauptsache aus einem Kunststoff (PEEK) mit darin eingelegten metallischen Gewindeeinsätzen 9 aus Titan, welche die Plattenlöcher 3 bilden. Die Plattenlöcher 3 weisen bei dieser Ausführungsform vier Ausnehmungen 6 auf, die sich radial von der Lochachse 5 weg über den Bereich der Innenmantelfläche 4 hinaus erstrecken. Die Innenmantelfläche 4 wird in vier Mantelflächenabschnitte unterteilt. Die Ausnehmungen erstrecken sich über die gesamte Höhe der Knochenplatte 1 von der Oberseite 8 bis zur Unterseite 2 hin konisch. Die Innenmantelfläche 4 ist zudem mit einer dreidimensionalen Strukturierung 7 in Form eines mehrgängigen Gewindes versehen.

Diese Ausführungsform kann materialmäßig auch invertiert werden, indem die Knochenplatte zur Hauptsache aus Metall (Titan) besteht mit darin eingelegten Gewindeeinsätzen 9 aus Kunststoff (PEEK), welche die Plattenlöcher 3 bilden.

In Fig. 6 ist die Knochenplatte nach Fig. 1 mit von oben eingesetzten Knochenschrauben 10 dargestellt, deren Kopfteil 11 sphärisch ausgebildet ist.

Fig. 7 zeigt die gleiche Knochenplatte 1 von unten.

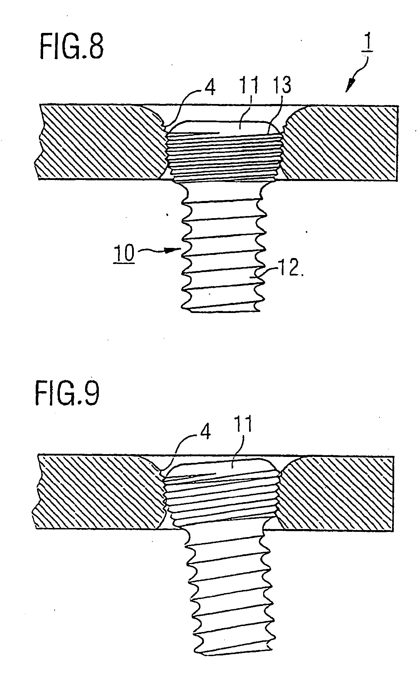

In Fig. 8 ist eine Knochenplatte 1 mit darin eingesetzter Knochenschraube 10 ohne Abwinkelung dargestellt. Die Innenmantelfläche 4 des Plattenlochs der Knochenplatte 1 und der Kopfteil 11 der Knochenschraube 10 weisen aufeinander abgestimmte Gewinde 13 auf.

In Fig. 9 ist die gleich Variante wie in Fig. 8 dargestellt, wobei jedoch die Knochenschraube 10 abgewinkelt ist.

## Patentansprüche

1. Bohrbüchse zur Verwendung in einer Knochenplatte (1) mit einer knochenseitigen Unterseite (2), einer Oberseite (8) und mehreren die Unterseite (2) mit der Oberseite (8) verbindenden Plattenlöchern (3), die jeweils eine zentrale Lochachse (5), eine sich gegen die Unterseite (2) hin verjüngendeInnenmantelfläche (4) und Gewindegänge, Rippen oder Erhebungen aufweisen, wobei die Innenmantelflächen (4) jeweils N≥3 sich radial von der Lochachse (5) weg erstreckende Ausnchmungen (6) aufweisen, welche die Gewindegänge, Rippen oder Erhebungen unterbrechen, **dadurch gekennzeichnet, dass** die kannulierte Bohrbüchse an ihrem zur Anlage in dem Plattenloch (3) der Knochenplatte (1) bestimmten Ende eine zur Geometrie der Innemantelfläche (4) des Plattenlochs (3) negative Geometrie aufweist, so dass die Bohrbüchse aufgrund der Ausnehmungen (6) in der Innenmantelfläche (4) des Plattenlochs (3) in das Plattenloch (3) einsteckbar ist.

## Claims

1. A drilling bush for use in a bone plate (1) having a bone-side underside (2), an upper side (8), and several plate holes (3) connecting the underside (2) to the upper side (8) and respectively having a central hole axis (5), an internal curved surface (4) tapering toward the underside (2), and thread turns, ribs or elevations, whereby the internal curved surfaces (4) respectively have N≥3 recesses (6) extending radially away from the hole axis (5) and interrupting the thread turns, ribs or elevations, **characterized in that** the cannulated drilling bush has, on its end intended for fitting in the plate hole (3) of the bone plate (1), a negative geometry with respect to the geometry of the internal curved surface (4) of the plate hole (3), so that the drilling bush is insertable into the plate hole (3) due to the recesses (6) in the internal curved surface (4) of the plate hole (3).

## Revendications

1. Douille de perçage destinée à être utilisée dans une plaque d'ostéosynthèse (1) comprenant une face de dessous (2) côté os, une face de dessus (8) et plusieurs trous de plaque (3) reliant la face de dessous (2) à la face de dessus (8) et présentant respectivement un axe de trou central (5), une surface d'enveloppe interne (4) s'effilant en direction de la face de dessous (2) et des pas de vis, des nervures ou des éminences, les surfaces d'enveloppe internes (4) présentant respectivement N≥3 évidements (6) s'étendant radialement à partir de l'axe (5) de trou et interrompant les pas de vis, nervures ou éminences, **caractérisée en ce que** la douille de perçage canulée présente à son extrémité destinée à être placée dans le trou de plaque (3) de la plaque d'ostéosynthèse (1) une géométrie négative par rapport à la géométrie de la surface d'enveloppe interne (4) du trou de plaque (3), de telle manière que la douille de perçage est enfichable dans le trou de plaque (3) en raison des évidements (6) se trouvant dans la surface d'enveloppe interne (4) du trou de plaque (3).
